# EUROPÄISCHE PATENTSCHRIFT

(11) **EP 3 377 640 B1**
(45) Veröffentlichungstag und Bekanntmachung des Hinweises auf die Patenterteilung: **21.12.2022**
(21) Anmeldenummer: 16797903.8
(22) Anmeldetag: 17.11.2016
(51) Int. Cl.: C12P 13/04

(54) **VERFAHREN ZUR HERSTELLUNG VON AMINOBENZOESÄURE ODER EINES AMINOBENZOESÄUREDERIVATS**
METHOD FOR THE PREPARATION OF AMINOBENZOIC ACID OR AN AMINOBENZOIC ACID DERIVATIVE
PROCEDE DE FABRICATION D'ACIDE AMINOBENZOÏQUE OU D'UN DERIVE D'ACIDE AMINOBENZOÏQUE

(30) Priorität: 20.11.2015 EP 15195527
(43) Veröffentlichungstag der Anmeldung: 26.09.2018
(73) Patentinhaber: Covestro Intellectual Property GmbH & Co. KG, 51373 Leverkusen (DE)
(72) Erfinder: JÄGER, Gernot, 50733 Köln (DE); BECKMANN, Wolfgang, 13465 Berlin (DE); MOUSSA, Amgad-Salah, 79713 Bad Säckingen (DE); OLF, Guenter, 40789 Monheim (DE); BECKER, Guido, 47800 Krefeld (DE); AKYILDIZ, Hasan, 47228 Duisburg (DE); DAMM, Andreas, 42929 Wermelskirchen (DE); HAMEDINGER, Thomas, 51375 Leverkusen (DE); KLOECKNER, Wolf, 51065 Köln (DE); BEHNKEN, Swantje, Sacramento, CA 95864 (US)
(74) Vertreter: Levpat
(86) Internationale Anmeldenummer: PCT/EP2016/077963
(87) Internationale Veröffentlichungsnummer: WO 2017/085170

(56) Entgegenhaltungen:
- EP-A1- 2 130 924
- WO-A1-2015/124687
- JP-A- H04 330 290
- SANDRA GRACIN ET AL: "Polymorphism and Crystallization of p -Aminobenzoic Acid", CRYSTAL GROWTH & DESIGN., Bd. 4, Nr. 5, September 2004 (2004-09), Seiten 1013-1023, XP055264738, US ISSN: 1528-7483, DOI: 10.1021/cg049954h

## Beschreibung

Die vorliegende Erfindung befasst sich mit einem Verfahren zur Herstellung von ortho-Aminobenzoesäure oder eines ortho-Aminobenzoesäurederivats durch Fermentation eines geeigneten Rohstoffes unter dem Einfluss geeigneter Mikroorganismen unter Erhalt einer ortho-Aminobenzoat und/oder ortho-Aminobenzoesäure umfassenden Fermentationsbrühe. Insbesondere befasst sich die vorliegende Erfindung mit dem Schritt der Gewinnung der ortho-Aminobenzoesäure aus der Fermentationsbrühe, wobei die Kristallisation von ortho-Aminobenzoesäure durch eine bloße einstufige Säurebehandlung in Gegenwart von Impfkristallen durchgeführt wird und die Anwesenheit der Impfkristalle derart erreicht wird, dass die Fermentationsbrühe und die Säure zu einer im Reaktor vorgelegten Suspension an Impfkristallen gegeben werden, wobei die in der Suspension vorgelegten Impfkristalle zu mindestens 90 %, bezogen auf die Gesamtmasse aller in der Suspension vorgelegten Impfkristalle, aus der Modifikation Form I der ortho-Aminobenzoesäure bestehen. Die so auf einfache Weise auskristallisierte ortho-Aminobenzoesäure kann leicht aus der Mutterlauge abgetrennt, erforderlichenfalls weiter gereinigt und dann den verschiedensten Anwendungszwecken zugeführt werden.

Die fermentative Herstellung von Aminobenzoesäure bzw. von Produkten, die durch weitere chemische Umwandlung von Aminobenzoesäure erhalten werden können (nachfolgend *Aminobenzoesäurederivate*) ist in der Literatur beschrieben. Für die fermentative Herstellung von Aminobenzoesäure sei beispielhaft auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen; siehe beispielsweise WO 2015/124686 A1 und WO 2015/124687 A1 sowie die darin jeweils zitierte Literatur. Produkte, die durch weitere chemische Umwandlung von Aminobenzoesäure erhalten werden können, werden von Wiklund *et al.* beschrieben (Per Wiklund et al., Current Organic Synthesis, 2006, 3, 379 - 402).

Auch andere Säuren wie L-Aminosäuren oder Nukleinsäuren wurden bereits fermentativ hergestellt, wie in EP 2 130 924 A1 beschrieben. In dem dort beschriebenen Verfahren fällt das gewünschte Produkt bereits in der Fermentationsbrühe aus, sodass auf einen separaten Kristallisationsschritt verzichtet werden kann. Optional werden *während der Fermentation* Impfkristalle zugegeben.

In S. Gracin et al., Crystal Growth & Design, 2004, 4, 1013 - 1023 werden der Polymorphismus und die Kristallisation von para-Aminobenzoesäure diskutiert. Die Schrift geht nicht auf Besonderheiten bei der Isolierung von para-Aminobenzoesäure *aus Fermentationsbrühen* ein. Die Isolierung einer Zielverbindung aus einer Fermentationsbrühe ist aber alles andere als trivial. Sie kann eine Reihe von Schritten wie Extraktion, Filtration, Adsorption oder Kristallisation umfassen. Es ist sogar möglich, dass *alle* diese Schritte erforderlich sind, um die gewünschte Zielverbindung in ausreichender Reinheit zu erhalten. Jeder dieser Schritte verursacht jedoch unweigerlich Mehraufwand und damit Kosten. Außerdem ist die Ausbeute an der Zielverbindung umso geringer, je mehr Schritte zu ihrer Isolierung erforderlich sind. Daher ist es generell wünschenswert, die Zahl der Schritte bis zur Isolierung der Zielverbindung in der gewünschten Reinheit möglichst klein zu halten.

Fermentationsprozesse laufen in der Regel in einer wässrigen Umgebung ab. Es ist daher insbesondere wünschenswert, die durch Fermentation hergestellte Zielverbindung direkt aus dieser wässrigen Umgebung isolieren zu können und etwa auf ein Herauslösen mittels eines organischen Lösungsmittels verzichten zu können.

Es ist allgemein bekannt, dass die Löslichkeit von ortho-Aminobenzoesäure als amphoterer Verbindung durch gezielte Einstellung des pH-Werts minimiert werden kann. Die beiden pKs-Werte liegen bei 2,2 bzw. 4,9 (vgl. Zapala et al., Biophys. Chem., 140 (1-3) (2009) 91 - 98), was mit einem Löslichkeitsminimum bei einem pH-Wert von etwa 3,5 korrespondiert.

Es wäre daher eigentlich zu erwarten, dass die Isolierung von ortho-Aminobenzoesäure aus Fermentationsbrühen ohne großen Aufwand möglich sein sollte. Im vollkommenen Gegensatz dazu beschreibt JP04-330290A einen mehrstufigen Prozess zur Isolierung von ortho-Aminobenzoesäure, in welchem
(1) optional und bevorzugt, durch eine pH-Wert-Anpassung der Fermentationsbrühe auf einen pH-Wert von 4 bis 6 in der Fermentationsbrühe vorhandene Proteine ausgefällt werden,
(2) die Biomasse aus der Fermentationsbrühe abgetrennt (und diese somit "sterilisiert") wird, dann
(3) durch eine pH-Wert-Anpassung auf einen pH-Wert von 5 bis 10 und anschließende Adsorption an einer geeigneten Säule eine Entfärbung bewirkt wird,
(4) die ortho-Aminobenzoesäure enthaltende Lösung aufkonzentriert wird, bevor
(5) auch nur damit begonnen werden kann, ortho-Aminobenzoesäure durch pH-Wert-Anpassung auf den dem isoelektrischen Punkt von ortho-Aminobenzoesäure entsprechenden pH-Wert auszukristallisieren, wonach schließlich
(6) die ausgefallenen Kristalle abgetrennt werden.

Das Verfahren umfasst also zwei bis drei Schritte der pH-Wert-Anpassung und ist daher im Hinblick auf die zur Kristallisation erforderliche Einstellung des pH-Werts mehrstufig. Darüber hinaus weist die aufwändig in fünf bis sechs Schritten isolierte ortho-Aminobenzoesäure nur eine Reinheit von 94 % auf und wird auch nur in einer relativ niedrigen Ausbeute von 67 % der theoretisch möglichen Ausbeute erhalten. Das Verfahren ist komplex und erfordert eine zwei- bis dreimalige pH-Wert-Änderung, was naturgemäß mit einer erhöhten Salzfracht der Abwässer einhergeht. Außerdem ist ortho-Aminobenzoesäure temperaturempfindlich und kann zum Aminobenzoesäurederivat Anilin decarboxylieren. Letzteres ist selbst dann problematisch, wenn Anilin das eigentliche Zielprodukt der Synthesesequenz ist, denn eine vorzeitige Decarboxylierung an dieser Stelle kann zu Ausbeuteverlusten infolge einer vergleichsweise hohen Wasserlöslichkeit von Anilin bei den relevanten pH-Werten führen. Eine möglichst rasche Isolierung der ortho-Aminobenzoesäure bei möglichst niedriger Temperatur ist daher wünschenswert.

WO 2015/124687 A1 offenbart auf S. 17 die Gewinnung von ortho-Aminobenzoesäure (Anthranilsäure) aus einer Fermentationsbrühe durch Säurezugabe in allgemeiner Weise, lehrt jedoch keine Details zur genauen Ausgestaltung dieses Schrittes.

Es bestand daher ein Bedarf an weiteren Verbesserungen in der fermentativen Herstellung von Aminobenzoesäure bzw. Aminobenzoesäurederivaten, und zwar insbesondere bei dem Schritt der Isolierung der Aminobenzoesäure aus der Fermentationsbrühe.

Diesem Bedarf Rechnung tragend ist ein Gegenstand der vorliegenden Erfindung ein **Verfahren zur Herstellung von ortho-Aminobenzoesäure oder eines eines Derivats des ortho-Isomers der Aminobenzoesäure,** umfassend die Schritte:
(I) **Fermentation** eines Rohstoffes, der wenigstens eine fermentierbare Kohlenstoff-haltige Verbindung umfasst und der bevorzugt ausgewählt ist aus der Gruppe bestehend aus Stärkehydrolysat, Zuckerrohrsaft und Zuckerrübensaft, unter Verwendung von Mikroorganismen einer Art ausgewählt aus der Gruppe bestehend aus *Escherichia coli, Pseudomonas putida* und *Corynebacterium glutamicum,* wobei eine ortho-Aminobenzoat und/oder ortho-Aminobenzoesäure umfassende Fermentationsbrühe erhalten wird;
(II) optionale **Vorbehandlung** der in Schritt (I) erhaltenen Fermentationsbrühe umfassend
   (1) **Abtrennung des Mikroorganismus** aus der in Schritt (I) erhaltenen Fermentationsbrühe ohne pH-Wert-Anpassung, wobei eine an Mikroorganismen abgereicherte Fermentationsbrühe erhalten wird,
      und/oder
   (2) **Entfärbung** der in Schritt (I) erhaltenen Fermentationsbrühe oder, bei Durchführung von Schritt (II) (1), der in Schritt (II) (1) erhaltenen an Mikroorganismen abgereicherten Fermentationsbrühe, ohne pH-Wert-Anpassung;
(III) **einstufige Behandlung** der in Schritt (I) oder Schritt (II) (1) oder Schritt (II) (2) erhaltenen Fermentationsbrühe in einem Reaktor **mit Säure** so, dass sich ortho-Aminobenzoesäure aus der Fermentationsbrühe abscheidet;
(IV) **Isolierung** der in Schritt (III) abgeschiedenen ortho-Aminobenzoesäure, wobei Mutterlauge zurückbleibt;
(V) optionale **weitere Reinigung** der in Schritt (IV) gewonnenen ortho-Aminobenzoesäure, bevorzugt durch Waschen mit Wasser;
(VI) optionale **weitere Umsetzung** der in Schritt (IV) oder Schritt (V) erhaltenen ortho-Aminobenzoesäure **zu einem Aminobenzosäurederivat;**
wobei Schritt (III) so durchgeführt wird, dass Impfkristalle von ortho-Aminobenzoesäure zugegen sind und die Anwesenheit der Impfkristalle derart erreicht wird, dass die Fermentationsbrühe und die Säure zu einer im Reaktor vorgelegten Suspension an Impfkristallen gegeben werden, wobei die in der Suspension vorgelegten Impfkristalle zu mindestens 90 %, bezogen auf die Gesamtmasse aller in der Suspension vorgelegten Impfkristalle, aus der Modifikation Form I der ortho-Aminobenzoesäure bestehen.

Die Säurebehandlung in Schritt (III) ist dabei "*einstufig*" in dem Sinne, dass der gewünschte Ziel-pH-Wert durch Säurezugabe direkt eingestellt wird, ohne dass bei pH-Werten zwischen dem Ausgangs-pH-Wert (d. i. der pH-Wert der in Schritt (I) oder Schritt (II) (1) oder Schritt (II) (2) erhaltenen Fermentationsbrühe, je nachdem, ob der optionale Schritt (II) durchgeführt wird und falls ja, aus welchen Teilschritten dieser besteht) und dem Ziel-pH-Wert (d. i. der pH-Wert, der sich nach beendeter Säurebehandlung in Schritt (III) einstellt) Zwischenschritte (wie Filtration, Zentrifugation, säulenchromatographische Behandlung und dgl.) durchgeführt werden. Die Einstellung des gewünschten Ziel-pH-Wertes durch die beschriebene einstufige Säurebehandlung erfolgt daher *nur* in Schritt (III) des erfindungsgemäßen Verfahrens. Die in Schritt (I) erhaltene Fermentationsbrühe wird also erfindungsgemäß entweder unmittelbar dem Schritt (III) unterworfen oder sie wird unmittelbar dem Schritt (II) unterworfen, und das Verfahrensprodukt des Schrittes (II) (d. h. das in Schritt (II) (1) oder Schritt (II) (2) erhaltene Verfahrensprodukt) wird dann unmittelbar dem Schritt (III) unterworfen. "*Unmittelbar"* bedeutet dabei *"ohne Zwischenschritte*". Mit anderen Worten: Schritt (II) *besteht aus* Schritt (II) (1) und/oder Schritt (II) (2). Die Schritte (II) (1) und (II) (2) werden erfindungsgemäß *"ohne pH-Wert-Anpassung",* d. h. ohne Säurebehandlung, durchgeführt. Die Vielzahl der im Stand der Technik beschriebenen pH-Wert-Anpassungen (Säurebehandlungen) wird im erfindungsgemäßen Verfahren daher auf eine einzige pH-Wert-Anpassung (Säurebehandlung) reduziert.

Als *"Impfkristalle* " im Sinne der vorliegenden Erfindung werden
(i) im Reaktor aus Schritt (III) *vorgelegte Kristalle von ortho-Aminobenzoesäure* (die auch aus einer externen Quelle stammen, z. B. zugekauft sein können) und
(ii) im Reaktor aus Schritt (III) *bei kontinuierlicher Durchführung dieses Schritts in situ gebildete Kristalle von ortho-Aminobenzoesäure, die als Impfkristalle für die Abscheidung weiterer ortho-Aminobenzoesäure dienen* (sog. Sekundärkeimbildung), wie weiter unten noch näher erläutert wird,
   aufgefasst.

Der Begriff *"ortho-Aminobenzoesäurederivat"* bezeichnet im Rahmen der vorliegenden Erfindung ein Produkt, das durch weitere chemische Umsetzung von ortho-Aminobenzoesäure erhalten wird. Das erfindungsgemäße Verfahren ermöglicht es, ortho-Aminobenzoesäure auf einfache Weise in hoher Reinheit zu gewinnen. In der Regel ist es nicht erforderlich, die gewonnene ortho-Aminobenzoesäure vor ihrer weiteren Verwendung umzukristallisieren; bevorzugt umfasst der Schritt (V), sofern durchgeführt, daher keine Umkristallisation. Die im Rahmen des erfindungsgemäßen Verfahrens nach Schritt (IV) verbleibende Mutterlauge enthält ortho-Aminobenzoesäure nur in geringen, bevorzugt die thermodynamische Löslichkeit nicht überschreitenden Mengen. Hierdurch wird die Abwasserbelastung minimiert. Das erfindungsgemäße Verfahren erlaubt ferner die Gewinnung von ortho-Aminobenzosäure in solchen Kristallgrößen, dass sich nach Abtrennung der Mutterlauge in Schritt (IV) bzw., wenn durchgeführt, nach Abtrennung des Waschwassers in Schritt (V), ein Restfeuchtigkeitsgehalt einstellt, der auch ohne aufwändige Trocknungsprozesse eine weitere Verwendung der ortho-Aminobenzoesäure ermöglicht.

Ausführungsformen der Erfindung werden nachfolgend näher beschrieben. Dabei können verschiedene Ausführungsformen beliebig miteinander kombiniert werden, sofern sich für den Fachmann aus dem Gesamtzusammenhang nicht das Gegenteil ergibt.

**Schritt (I)** des erfindungsgemäßen Verfahrens kann nach einer beliebigen aus dem Stand der Technik bekannten Verfahrensweise durchgeführt werden. Besonders bevorzugt sind Verfahren, wie sie in WO 2015/124686 A1 und WO 2015/124687 A1 unter Verwendung von Bakterien als Mikroorganismen beschrieben sind. Dabei wird insbesondere auf WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31) verwiesen.

Es versteht sich von selbst, dass zur fermentativen Herstellung von ortho-Aminobenzoesäure neben einer Kohlenstoff- auch eine Stickstoffquelle erforderlich ist. Sofern die in Schritt (I) einzusetzende fermentierbare Kohlenstoff-haltige Verbindung nicht bereits eine geeignete Stickstoff-haltige Verbindung als Stickstoffquelle enthält, ist eine solche zuzusetzen. Bevorzugt wird zu diesem Zweck die Stickstoff-haltige Verbindung ausgewählt aus der Gruppe bestehend aus Ammoniakgas, Ammoniakwasser, Ammoniumsalzen (insbesondere Ammoniumsulfat und Ammoniumchlorid) und Harnstoff.

Erfindungsgemäß umfassen die in Schritt (I) eingesetzten Mikroorganismen eine Art ausgewählt aus der Gruppe bestehend aus *Escherichia coli, Pseudomonas putida* und *Corynebacterium glutamicum.* Besonders bevorzugt bestehen die Mikroorganismen nur aus Vertretern genau einer dieser Arten. *Corynebacterium glutamicum* ATTC 13032 ist dabei insbesondere bevorzugt.

Der in der Fermentation einzuhaltende pH-Wert richtet sich nach dem eingesetzten Mikroorganismus. Die Fermentation in Schritt (I) wird insbesondere bei einem solchen pH-Wert durchgeführt, dass eine spontane Abscheidung von ortho-Aminobenzoesäure bereits in der Fermentationsbrühe weitgehend bis vollständig vermieden wird, d. h. Schritt (I) wird insbesondere bei einem pH-Wert von ≥ 6,5 durchgeführt. Mikroorganismen wie *Corynebacterium glutamicum, Pseudomonas putida* oder *Escherichia coli* werden bevorzugt bei im Wesentlichen neutralen pH-Werten (d. h. bevorzugt bei einem pH-Wert im Bereich von 6,5 bis 8,0) kultiviert.

In jedem Fall wird der Mikroorgansimus aus Schritt (I) erfindungsgemäß so ausgewählt, dass in der Fermentation (selektiv) das ortho-Isomer von Aminobenzoesäure und/oder Aminobenzoat gebildet wird. *"Selektiv"* bedeutet dabei, dass andere Isomere nicht oder allenfalls in untergeordneten Anteilen (d. h. in Anteilen - wie bestimmt per Hochleistungsflüssigchromatographie, HPLC - von insgesamt maximal 0,50 %, bevorzugt maximal 0,25 %, ganz besonders bevorzugt maximal 0,10 %, außerordentlich ganz bevorzugt maximal 0,05 %, jeweils bezogen auf die Gesamtheit aller Aminobenzoesäure-Isomeren) gebildet werden. Hierzu eignen sich insbesondere Mikroorganismen einer Art ausgewählt aus der Gruppe bestehend aus *Escherichia coli, Pseudomonas putida* und *Corynebacterium glutamicum.* Im Folgenden ist mit den Ausdrücken "Aminobenzoesäure" und "Aminobenzoat" auch dann das ortho-Isomer gemeint, wenn dies nicht ausdrücklich gesagt wird.

Um ein geeignetes Bakterium zu erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der Bakterienzelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von Bakterien mit den zuvor genannten Eigenschaften sind im Stand der Technik bekannt. Geeignete Bakterien können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

Besonders bevorzugt enthalten die in Schritt (I) einzusetzenden Bakterien eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht dabei sich auf eine Enzymaktivität, durch welche die Umsetzung von ortho-Aminobenzoat zu N-(5-Phospho-D-Ribosyl)-Anthranilat katalysiert wird.

In dem Bakterium *Corynebacterium glutamicum* wird die Anthranilat-Phosphoribosyltransferase-Aktivität durch das *trpD* Gen (cg3361, Cgl3032, NCgl2929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. *trpD* oder *trpDC*), kodiert werden.

Unabhängig davon, welcher Mikroorganismus eingesetzt wird, umfasst die Fermentationsbrühe zu Beginn der Fermentation in Schritt (I) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle, entweder als Bestandteil der Kohlenstoff-haltigen Verbindung oder zugesetzt). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure und/oder Aminobenzoat (abhängig vom pH-Wert, bei dem die Fermentation durchgeführt wird), das angestrebte Fermentationsprodukt.

Unter einer fermentierbaren Kohlenstoff-haltigen Verbindung im Sinne der vorliegenden Erfindung wird jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden.

Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen. Ebenfalls geeignet sind Glycerin und C1-Verbindungen, insbesondere Kohlenstoffmonoxid.

In einer bevorzugten Ausführungsform wird Schritt (I) kontinuierlich durchgeführt, d. h. dass die Edukte dem Fermentationsreaktor kontinuierlich zugeführt und das Produkt dem Fermentationsreaktor kontinuierlich entnommen wird. In kontinuierlicher Verfahrensführung wird der Mikroorganismus unter Umständen mit dem Produktstrom ausgetragen; der Mikroorganismus reproduziert sich jedoch im Allgemeinen selbst, sodass eine Zuführung von frischem Mikroorganismus in der Regel nicht nötig ist (erforderlichenfalls aber natürlich vorgenommen werden kann). Eine Zellrückhaltung zur Vermeidung der Austragung von Mikroorganismus ist ebenfalls möglich.

In einer anderen bevorzugten Ausführungsform wird Schritt (I) in einer diskontinuierlichen Verfahrensführung (sog. "Batch-Fahrweise") durchgeführt. In einer Variante der diskontinuierlichen Fahrweise (sog. "*Fed*-Batch-Fahrweise") werden die Edukte dem Fermentationsreaktor so lange kontinuierlich oder in Intervallen, bevorzugt kontinuierlich, zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Fermentationsreaktor entnommen.

Unabhängig von der genauen Fahrweise umfasst der Reaktionsapparat, in welchem Schritt (I) durchgeführt wird (im Folgenden *Fermentationsreaktor*)*,* bevorzugt Einrichtungen zur Messungen wichtiger Prozessparameter wie Temperatur, pH-Wert der Fermentationsbrühe, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren ("plug flow reactors") oder Schlaufenreaktoren (siehe beispielsweise Bioprozesstechnik, Horst Chmiel, ISBN-10: 3827424763, Spektrum Akademischer Verlag). Besonders bevorzugt sowohl für aerobe als auch für anaerobe Fermentationen sind Rührkesselreaktoren und Schlaufenreaktoren (insbesondere Airliftreaktoren, in denen die Zirkulation der Flüssigkeit im Reaktor durch Begasung erreicht wird).

Der optionale **Schritt (II) (1),** die Abtrennung des Mikroorganismus aus der Fermentationsbrühe, ist an sich aus dem Stand der Technik bekannt und erfolgt im Rahmen der vorliegenden Erfindung insbesondere durch Filtration oder Zentrifugation. Bevorzugt wird dieser Schritt, sofern er erfolgt, durchgeführt wie in WO 2015/124686 A1 und WO 2015/124687 A1 beschrieben. Dabei wird insbesondere auf WO 2015/124687 A1, Seite 15, Zeile 8 bis Seite 15, Zeile 17, verwiesen.

Der optionale **Schritt (II) (2),** der sich, wenn durchgeführt, entweder an Schritt (I) oder an Schritt (II) (1) anschließt, wird bevorzugt so durchgeführt, dass Fermentationsbrühe bzw. an Mikroorganismen abgereicherte Fermentationsbrühe über eine Säule mit fester Packung geleitet wird, um Farbstoffe mittels Adsorption zu entfernen. Als mögliche feste Phase kann z. B. Kieselgur oder Ionenaustauscherpackungen verwendet werden. Schritt (II) (2) wird bevorzugt dann durchgeführt, wenn in der Fermentationsbrühe aus Schritt (I) oder (II) (1) solche farbigen Substanzen vorhanden sind, die die nachfolgende Kristallisation stören könnten.

In **Schritt (III)** des erfindungsgemäßen Verfahrens wird durch Säurezugabe zur Fermentationsbrühe der pH-Wert so eingestellt, dass Aminobenzoesäure auskristallisiert. Diese Art der Kristallisation wird auch als *Reaktiv*kristallisation bezeichnet. Dies geschieht bevorzugt derart, dass der pH-Wert der resultierenden Mischung dem des isoelektrischen Punkts des ortho-Isomers von Aminobenzoesäure entspricht oder diesem zumindest nahekommt. Daher wird der pH-Wert bevorzugt auf einen Wert im Bereich von 3,0 bis 4,7, besonders bevorzugt auf einen Wert im Bereich von 3,2 bis 3,7, ganz besonders bevorzugt auf einen Wert im Bereich von 3,4 bis 3,6, eingestellt, also nahe oder entsprechend dem isoelektrischen Punkt bei pH 3,5.

Als Säure kommen alle Säuren in Betracht, mit denen ein pH-Wert, der dem isolektrischen Punkt des ortho-Isomers der Aminobenzoesäure entspricht oder zumindest nahekommt, eingestellt werden kann. Bevorzugt werden hierzu starke Mineralsäuren, insbesondere Salzsäure, Schwefelsäure und/oder Phosphorsäure eingesetzt. Bevorzugt umfasst die in Schritt (III) eingesetzte Säure Salzsäure, besonders bevorzugt Salzsäure einer Konzentration von 15 Massen-% bis 37 Massen-%, ganz besonders bevorzugt Salzsäure einer Konzentration von 18 Massen-% bis 25 Massen-%. Es ist insbesondere bevorzugt, dass die Säure neben dieser Salzsäure mit Ausnahme von optional zugesetzter rezyklierter Mutterlauge aus Schritt (IV) keine weitere Säure umfasst (d. h. es wird keine weitere Säure aus einer externen Quelle zugegeben). Wenn als in Schritt (III) eingesetzte Säure eine Mischung aus Salzsäure und einem Teil der in Schritt (IV) erhaltenen Mutterlauge eingesetzt wird, so werden bevorzugt 1,0 Massen-% bis 50 Massen-% der in Schritt (IV) insgesamt erhaltenen Mutterlauge mit Salzsäure vermischt.

Als *Reaktor* in Schritt (III) kommen dem Fachmann geläufige übliche Ausgestaltungen chemischer Reaktoren in Betracht. Beispielhaft seien Rührkessel oder Zwangsumlaufkristallisatoren wie solche vom "Typ Oslo" genannt. Mögliche Reaktoren für Schritt (III) (auch "Kristaller" genannt) sind in den beigefügten Zeichnungen dargestellt:

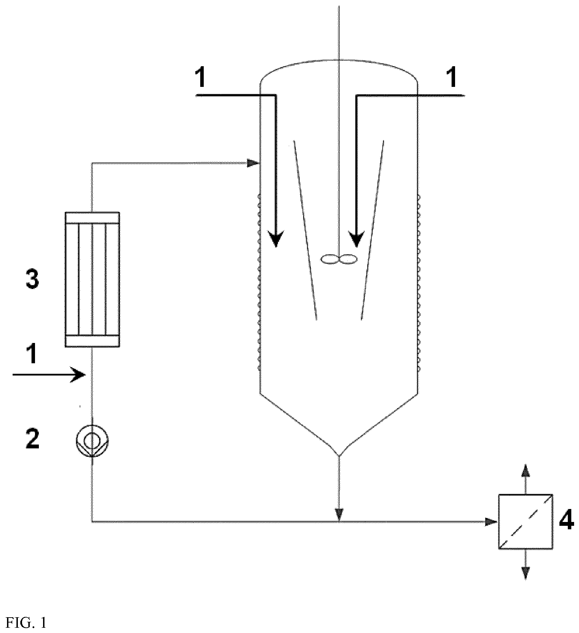

**FIG. 1** zeigt einen Kristaller mit Zwangsumlaufkristallisation ohne Einbauten. Es bedeuten: (1) Mögliche Zufuhreinrichtungen für Fermentationsbrühe bzw. Säure, (2) Pumpe bzw. Crusher, (3) Wärmeaustauscher, (4) Fest-Flüssigtrennung (z. B. Filtration).

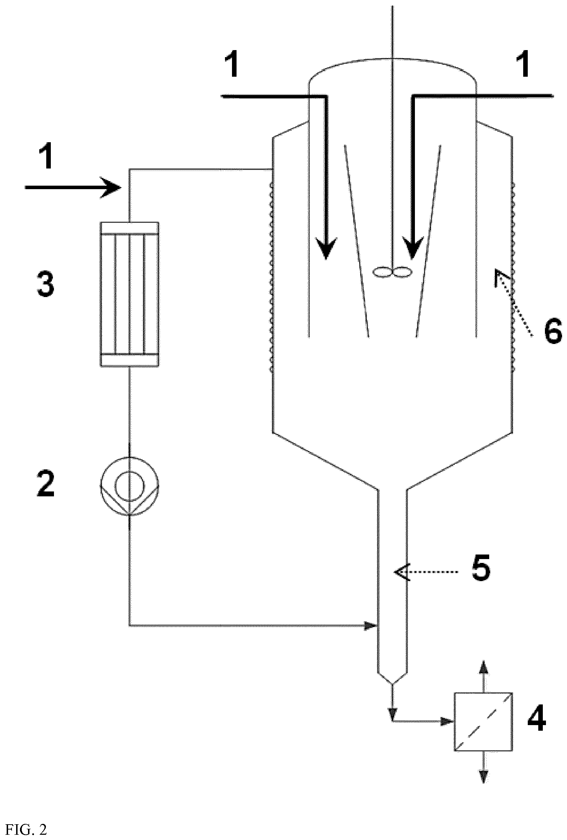

**FIG. 2** zeigt einen Kristaller mit Zwangsumlaufkristallisation und Einbauten, wobei der Umpumpkreislauf seitlich des Sichters eingesetzt wird. Es bedeuten: (1) Mögliche Zufuhreinrichtungen für Fermentationsbrühe bzw. Säure, (2) Pumpe bzw. Crusher, (3) Wärmetauscher, (4) Fest-Flüssigtrennung (z. B. Filtration), (5) Sichter, (6) Beruhigungszone.

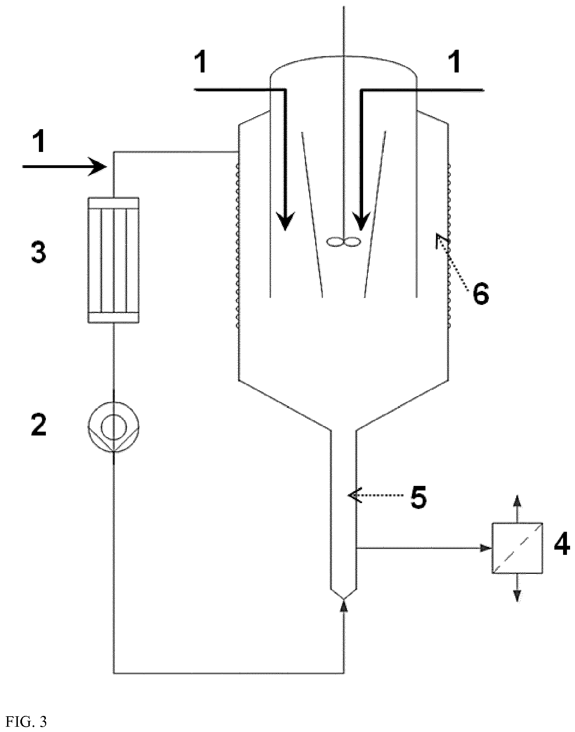

**FIG. 3** zeigt einen Kristaller mit Zwangsumlaufkristallisation und Einbauten, wobei der Umpumpkreislauf am Sichterboden zur Aufwirbelung eingesetzt wird. Es bedeuten: (1) Mögliche Zufuhreinrichtungen für Fermentationsbrühe bzw. Säure, (2) Pumpe bzw. Crusher, (3) Wärmetauscher, (4) Fest-Flüssigtrennung (z. B. Filtration), (5) Sichter, (6) Beruhigungszone.

Es hat sich bewährt, in Schritt (III) die Fermentationsbrühe und die Säure dem Reaktor über räumlich (möglichst weit) getrennte Zuführeinrichtungen zuzuführen. Hierdurch wird erreicht, dass die Reaktanden möglichst gut mit dem Reaktorinhalt vermischt werden, bevor es zur Säure-Base-Reaktion kommt (vgl. z. B. Beckmann, Crystallization, Wiley 2013, S. 175 bis 176). Als Zuführeinrichtungen kommen bspw. Rohrleitungen, bevorzugt mit Absperrventilen, in Betracht. In einer Ausführungsform sind die Zuführeinrichtung für die Fermentationsbrühe und die Zuführeinrichtung für die Säure an gegenüberliegenden Stellen der Reaktorwandung (im Wesentlichen) rechtwinklig zu dieser angeordnet. In einer anderen Ausführungsform sind die Zuführeinrichtung für die Fermentationsbrühe und die Zuführeinrichtung für die Säure (im Wesentlichen) parallel zur Reaktorwandung angeordnet, wobei sich die Zuführeinrichtungen gegenüberliegen und möglichst nahe, insbesondere unmittelbar, an der Reaktorwand befinden.

Es ist möglich, den in Schritt (III) eingesetzten Reaktor durch geeignete Einbauten in Kammern zu unterteilen. Mögliche resultierende Kammern sind die in FIG. 2 und FIG. 3 dargestellten Beruhigungszonen (6) oder Sichter (5). Durch Wahl von Rührergeometrie und Rührerbetriebsweise kann die Strömungsrichtung eingestellt werden. Es ist ebenfalls möglich, den Reaktor mit einem externen Umpumpkreislauf zu versehen, wobei dann einer der beiden Reaktanden - Fermentationsbrühe oder Säure - in den Umpumpkreislauf und der andere direkt in den Reaktor gegeben wird (vgl. die Zeichnungen). Wird ein Reaktor mit Sichter und Umpumpkreislauf betrieben, wird der Umpumkreislauf am Sichterboden zur Aufwirbelung oder seitlich des Sichters eingesetzt.

Es hat sich ferner bewährt, in Schritt (III) bei kontinuierlicher Durchführung dieses Schritts
▪ die Säure mit einer solchen Dosiergeschwindigkeit und
▪ die Fermentationsbrühe mit einer solchen Dosiergeschwindigkeit
dem Reaktor zuzuführen und eine Suspension von Produkt (also in sauer gestellter Fermentationsbrühe [= Mutterlauge] suspendierte Aminobenzoesäure) dem Reaktor absatzweise oder kontinuierlich, bevorzugt kontinuierlich, so zu entnehmen, dass sich eine Verweilzeit der Suspension im Reaktor von ¼ h bis 10 h, bevorzugt von ¼ h bis 2 h, ergibt. Unter einer *kontinuierlichen Durchführung des Schrittes (III)* wird im Sinne der vorliegenden Erfindung eine Fahrweise verstanden, bei der dem Reaktor aus Schritt (III) Säure und Fermentationsbrühe kontinuierlich zugeführt und Produkt (also in sauer gestellter Fermentationsbrühe [= Mutterlauge] suspendierte Aminobenzoesäure) dem Reaktor zumindest absatzweise (*semi-kontinuierliche Fahrweise*) oder bevorzugt ebenfalls kontinuierlich (*vollkontinuierliche Fahrweise*) entnommen wird.

Bei einer diskontinuierlichen Durchführung von Schritt (III) ist es bevorzugt, die Säurebehandlung in Schritt (III) über einen Zeitraum von ¼ h bis 10h, bevorzugt von ¼ h bis 2 h, durchzuführen. Unter einer *diskontinuierlichen Durchführung des Schrittes (III)* wird im Sinne der vorliegenden Erfindung eine Fahrweise verstanden, bei der alle Reaktanden dem Reaktor zugeführt und dort für einen gewünschten Zeitraum zur Reaktion gebracht werden. Nach Beendigung des Schrittes (III) wird dann die abgeschiedene Aminobenzoesäure aus dem erhaltenen Reaktionsgemisch isoliert (Schritt (IV); siehe unten für Details). Die Gewinnung der Aminobenzoesäure erfolgt also in dieser Ausführungsform *chargenweise.*

Unabhängig von der Fahrweise (kontinuierlich oder diskontinuierlich) werden die genauen Betriebsparameter (u. a.) durch die gewünschte Kristallgröße bestimmt, welche durch die Verweilzeit/Reaktionszeit und den Übersättigungsgrad eingestellt werden kann (große Kristallgrößen werden durch lange Verweilzeiten/lange Reaktionszeiten und geringe Übersättigungsgrade begünstigt).

**Die Anwesenheit von Impfkristallen** von Aminobenzoesäure während der Säurebehandlung von Schritt (III) kann wie folgt realisiert werden:
Erfindungsgemäß wird die Anwesenheit von Impfkristallen von Aminobenzoesäure in Schritt (III) dadurch erreicht, dass die Fermentationsbrühe und die Säure zu einer im Reaktor vorgelegten Suspension an Impfkristallen gegeben werden. Diese Impfkristalle können aus einer früheren Produktionscharge oder einer externen Quelle stammen. Diese Vorgehensweisewird bei diskontinuierlicher Durchführung von Schritt (III) und bei der Inbetriebnahme einer kontinuierlich durchgeführten Abscheidung von Aminobenzoesäure in Schritt (III) des erfindungsgemäßen Verfahrens angewandt. Bevorzugt wird in beiden Fällen als Suspension eine Suspension der Impfkristalle in einem Teil der in Schritt (I) oder Schritt (II) (1) oder Schritt (II) (2) erhaltenen Fermentationsbrühe eingesetzt, wobei die Impfkristalle bevorzugt in 1,0 Massen-% bis 20 Massen-% der in Schritt (III) insgesamt eingesetzten Fermentationsbrühe suspendiert werden.

Bevorzugt wird Schritt (III) des erfindungsgemäßen Verfahrens kontinuierlich, wie zuvor definiert, durchgeführt. In dieser Ausführungsform es ist vorgesehen, zur Inbetriebnahme der kontinuierlich durchgeführten Abscheidung von Aminobenzoesäure durch Säurebehandlung in Schritt (III) des erfindungsgemäßen Verfahrens im Reaktor eine Suspension an Impfkristallen vorzulegen, zu welcher dann die Fermentationsbrühe und die Säure kontinuierlich zugegeben werden. Daraufhin beginnt Aminobenzoesäure sich abzuscheiden, wobei der pH-Wert als Stellgröße für die Abscheidungsgeschwindigkeit dienen kann. Sobald die Abscheidung von Aminobenzoesäure im Reaktor aus Schritt (III) begonnen hat, wirken die *in situ* abgeschiedenen Kristalle der Aminobenzoesäure als Impfkristalle für die Abscheidung weiterer Aminobenzoesäure. In dieser Ausführungsform der Erfindung wird die Anwesenheit von Impfkristallen - neben den zur Inbetriebnahme vorgelegten Impfkristallen - von Aminobenzoesäure in Schritt (III) also bevorzugt dadurch erreicht, dass Fermentationsbrühe und Säure kontinuierlich dem Reaktor zugeführt werden und eine Suspension von Aminobenzoesäure dem Reaktor absatzweise oder kontinuierlich, bevorzugt kontinuierlich, entnommen wird, wobei die Zufuhr an Fermentationsbrühe und Säure sowie die Entnahme an Suspension so eingestellt werden, dass in dem im Reaktor befindlichen Teil der Reaktionsmischung (also in dem Teil der Reaktionsmischung, der nicht gerade entnommen wird) stets Kristalle von Aminobenzoesäure, die als Impfkristalle wirken, vorhanden sind. Bevorzugt werden die Zufuhr an Fermentationsbrühe und Säure sowie die Entnahme an Suspension so eingestellt, dass die Menge an in einem Zeitraum über die ausgetragene Suspension entnommener Aminobenzoesäure der Menge an im selben Zeitraum neu abgeschiedener Aminobenzoesäure entspricht. Die für eine kontinuierliche Verfahrensweise erforderliche kontinuierliche Erzeugung von Kristallisationskeimen durch Sekundärkeimbildung kann durch jede der bekannten, dem Fachmann geläufigen Methoden erfolgen; zB. durch Stöße mit einem Rührorgan oder durch Umpumpen (vgl. auch Beckmann, Crystallization, Wiley 2013, S. 203 bis 233). Die Details hängen von der tatsächlichen Ausführung des Reaktors aus Schritt (III) (des "Kristallers") und der gewünschten Kristallgröße ab; ggf. ist eine Klassierung erforderlich. Eine Klassierung kann mit Hilfe eines Sichters oder Hydrozyklons erfolgen. Bei kontinuierlicher Verfahrensführung ist das Vorlegen von Impfkristallen aus einer externen Quelle - zugekauft oder aus einer früheren Produktionscharge - also nur bei der Inbetriebnahme erforderlich; es ist nicht nötig und nicht bevorzugt, während der kontinuierlich durchgeführten Abscheidung der Aminobenzoesäure permanent Impfkristalle aus einer solchen externen Quelle zuzuführen.

Unabhängig von der gewählten Ausführungsform wird der Anteil an in Suspension vorgelegten Impfkristallen, sofern vorhanden, in Schritt (III) im erfindungsgemäßen Verfahren bevorzugt auf einen Wert von 0,50 Massen-% bis 30 Massen-%, bezogen auf die Gesamtmasse der in Schritt (III) abzuscheidenden Aminobenzoesäure, eingestellt. Als *Gesamtmasse der in Schritt (III) abzuscheidenden Aminobenzoesäure* wird für diesen Zweck vereinfachend die Masse der Aminobenzoesäure bei 100%iger Abscheidung zugrunde gelegt. Diese ergibt sich in einfacher Weise aus den bekannten Einsatzkonzentrationen und -mengen.

Das erfindungsgemäße Verfahren wird erfindungsgemäß zur Herstellung von ortho-Aminobenzoesäure oder eines entsprechenden Aminobenzoesäurederivats eingesetzt. Es ist vorgesehen, dass die in der Suspension in Schritt (III) vorgelegten Impfkristalle zu mindestens 90 %, bevorzugt zu mindestens 95 %, bezogen auf die Gesamtmasse aller in der Suspension vorgelegten Impfkristalle, aus der Modifikation Form I bestehen, gemäß der in Ojala, W. H.; Etter, M. C., J. Am. Chem. Soc. 1992, 114, 10288 - 10293 beschriebenen Nomenklatur. Hierdurch wird eine bevorzugte Kristallisation dieser Modifikation bewirkt, was eine Ausbeuteerhöhung zur Folge hat.

**Schritt (IV),** die Isolierung der in Schritt (III) abgeschiedenen Aminobenzoesäure, ist an sich aus dem Stand der Technik bekannt und erfolgt erfindungsgemäß bevorzugt durch Filtration oder Zentrifugation. Bevorzugt wird dieser Schritt durchgeführt wie in WO 2015/124687 A1 beschrieben. Dabei wird insbesondere auf WO 2015/124687 A1, Seite 17, Zeile 13 bis Seite 17, Zeile 16, verwiesen. Die Filtration kann bei vermindertem Druck, Umgebungsdruck oder erhöhtem Druck durchgeführt werden. Bei kontinuierlicher Durchführung von Schritt (III) wird auch Schritt (IV) bevorzugt kontinuierlich durchgeführt, d. h. die in Schritt (III) absatzweise oder kontinuierlich entnommene Suspension wird direkt im Anschluss an Schritt (III) absatzweise oder kontinuierlich isoliert.

Der optionale **Schritt (V),** die weitere Reinigung der in Schritt (IV) gewonnenen Aminobenzoesäure, ist an sich aus dem Stand der Technik bekannt (siehe vor allem WO 2015/124687 A1 und insbesondere auf WO 2015/124687 A1, Seite 18, Zeile 4 bis Seite 18, Zeile 6) und erfolgt erfindungsgemäß bevorzugt durch eine oder mehrere Wäschen mit wässrigen Waschmedien, insbesondere Wasser. Um Ausbeuteverluste zu vermeiden, kann der pH-Wert des wässrigen Waschmediums auf den gleichen Wert wie in Schritt (III) nach beendeter Säurezugabe eingestellt werden; es wird also in dieser Ausführungsform statt mit Wasser mit einer verdünnten Säure, insbesondere der gleichen Säure wie in Schritt (III) eingesetzt, gewaschen. Das nach Entfernung des wässrigen Waschmediums durch Filtration, ggf. unterstützt durch Anlegen eines erniedrigten oder erhöhten Drucks, zurückbleibende Produkt weist im Allgemeinen, infolge großer Kristallgrößen, eine so geringe Restfeuchte auf, dass auf aufwändige weitere Trocknungsschritte vor der weiteren Verwendung verzichtet werden kann. Wird auf Schritt (V) verzichtet, gilt das Gleiche für das nach Schritt (IV) erhaltene Produkt.

Die erfindungsgemäß gewonnene Aminobenzoesäure wird bevorzugt zu einem *Aminobenzoesäurederivat* weiter umgesetzt, d. h. **Schritt (VI)** wird bevorzugt durchgeführt. Ausgewählte weitere Umsetzungen der erhaltenen Aminobenzoesäure in Schritt (VI) sind:
(VI-1) Decarboxlierung zu Anilin;
(VI-2) Decarboxylierung gefolgt von säurekatalysierter Reaktion mit Formaldehyd unter Bildung von Di- und Polyaminen der Diphenylmethanreihe;
(VI-3) Decarboxylierung gefolgt von säurekatalysierter Reaktion mit Formaldehyd, gefolgt von Umsetzung mit Phosgen unter Bildung von Di- und Polyisocyanaten der Diphenylmethanreihe;
(VI-4) Umsetzung zu einer Azoverbindung, insbesondere zu einem Azo-Farbstoff;
(VI-5) Umsetzung zu Amiden;
(VI-6) Umsetzung zu leitenden Polymeren wie insbesondere Polyanthranilsäure.

Die Decarboxylierung von ortho-Aminobenzoesäure zu **Anilin (VI-1)** ist an sich bekannt und kann im Rahmen der vorliegenden Erfindung genauso durchgeführt werden wie in der Literatur beschrieben. Geeignete Verfahrensweisen sind beispielsweise in WO 2015/124686 A1 und WO 2015/124687 A1 beschreiben. Die Decarboxylierung wird bevorzugt bei einer Temperatur im Bereich 150 °C bis 250 °C, besonders bevorzugt im Bereich von 160 °C bis 220 °C, ganz besonders bevorzugt im Bereich von 180 °C bis 200 °C, durchgeführt. Die Decarboxylierung kann rein thermisch bewirkt, aber auch katalytisch betrieben werden. Als Katalysatoren eignen sich bspw. Zeolithe.

Die weitere Umsetzung des so erhaltenen Anilins mit Formaldehyd zu **Di- und Polyaminen der Diphenylmethanreihe (VI-2)** ist ebenfalls an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe (VI-3)** ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung der erfindungsgemäß erhaltenen Aminobenzoesäure zu **Azoverbindungen, insbesondere zu Azo-Farbstoffen (VI-4)** kann nach einem beliebigen Verfahren des Standes der Technik erfolgen. Beispielhaft sei auf die bekannte Herstellung von Methylrot oder Indigo verwiesen (Per Wiklund et al., Current Organic Synthesis, 2006, 3, 379 - 402).

Die Umsetzung der erfindungsgemäß erhaltenen Aminobenzoesäure zu **Amiden (VI-5)** kann ebenfalls nach einem beliebigen Verfahren des Standes der Technik erfolgen. Beispielhaft sei das primäre Amin von Anthranilsäure (2-Aminobenzylamid) erwähnt, welches unter anderem als Startmaterial für die Herstellung von Pharmazeutika verwandt wird (Per Wiklund et al., Current Organic Synthesis, 2006, 3, 379 - 402).

Die Umsetzung zu leitenden Polymeren wie insbesondere Polyanthranilsäure ist beispielsweise beschrieben in Bhavana Guptaa et al., Polymers Advanced Technologies, 2011, 22, 1982-1988.

### Beispiele

In allen Beispielen wurde die jeweils eingesetzte Fermentationsbrühe hergestellt durch Fermentation von rekombinanten *Corynebacterium-glutamicum*-ATTC-13032-Stämmen, die eine Deletion bzw. reduzierte Expression des trpD Gens aufweisen, welches für die Anthranilat-Phosphoribosyltransferase kodiert - wie in WO 2015/124687 A1, insbesondere auf S. 6, Z. 8 bis 28 und Beispiel 3 beschrieben (entspricht **Schritt (I)** des erfindungsgemäßen Verfahrens). Zur Abreicherung des eingesetzten Mikroorganismus wurde die Fermentationsbrühe filtriert **(Schritt (II)** des erfindungsgemäßen Verfahrens).

### Beispiel 1 (Vergleich: diskontinuierliche Durchführung der Säurebehandlung - Zugabe von Salzsäure zu vorgelegter Fermentationsbrühe ohne Impfkristalle)

100 g einer Fermentationsbrühe mit einem Gehalt an ortho-Aminobenzoesäure von 91 g/L und einen pH-Wert von 6,9 wurden in einem Planschliffkessel vorgelegt und über 1 h mit Salzsäure mit einem Massenanteil von 10 % auf einen pH-Wert von 3,6 eingestellt. Hierzu waren 29,2 g der Salzsäure erforderlich, was einem Äquivalenzverhältnis von 1,1 entspricht (entspricht bis auf die fehlenden Impfkristalle **Schritt (III)** des erfindungsgemäßen Verfahrens).

Nach einer Nachrührzeit von 1 h wurde das Kristallisat durch Filtration isoliert **(Schritt (IV)** des erfindungsgemäßen Verfahrens) und mit Wasser gewaschen **(Schritt (V)** des erfindungsgemäßen Verfahrens).

Der Filterwiderstand lag bei α = 5,3 10⁺¹⁰ 1/m², die Restfeuchte im Kuchen lag bei 30,7 % und die isolierte Ausbeute betrug 88 %. Die Kristallgröße wurde anhand von mikroskopischen Bildern zu 200 µm abgeschätzt.

Das Kristallisat lag in der Modifikation II vor, was aufgrund von dessen im Vergleich zur Modifikation I höheren Löslichkeit nachteilig ist. Der Gehalt des Kristallisats an ortho-Aminobenzoesäure lag bei 93 %.

### Beispiel 2 (Vergleich: diskontinuierliche Durchführung der Säurebehandlung - Zugabe von Salzsäure zu vorgelegter Fermentationsbrühe ohne Impfkristalle)

In einer Variation von Beispiel 1 wurde eine 37%-ige Salzsäure eingesetzt.

Der Filterwiderstand wurde zu α = 5,0 10⁺¹⁰ 1/m² bestimmt, die Restfeuchte im Kuchen lag bei 61 % und die isolierte Ausbeute betrug 85 %. Die Kristallgröße wurde anhand von mikroskopischen Bildern zu 100 µm abgeschätzt.

Das Kristallisat lag in der Modifikation II vor. Der Gehalt des Kristallisats an ortho-Aminobenzoesäure lag bei 94 %, der Aschegehalt betrug 0,55 %.

### Beispiel 3 (Vergleich diskontinuierliche Durchführung der Säurebehandlung - Zugabe von Salzsäure zu vorgelegter Fermentationsbrühe ohne Impfkristalle)

In einer anderen Variation von Beispiel 1 erfolgte der Zusatz der Salzsäure über 5 min.

Der Filterwiderstand wurde zu α = 4,9 10⁺¹⁰ 1/m² bestimmt, die Restfeuchte im Kuchen lag bei 46,7 % und die isolierte Ausbeute betrug 93 %. Die Kristallgröße wurde anhand von mikroskopischen Bildern zu 150 µm abgeschätzt.

Das Kristallisat lag in der Modifikation II vor.

### Beispiel 4 (erfindungsgemäß: diskontinuierliche Durchführung der Säurebehandlung - kontinuierliche Zuführung von Salzsäure und Fermentationsbrühe zu einer Suspension an Impfkristallen)

Im sog. Fed-Batch-Verfahren wurden Salzsäure und Fermentationsbrühe kontinuierlich zu einer vorgelegten Suspension von Impfkristallen der Modifikation I in Fermentationsbrühe mit einem pH-Wert von 3,5 dosiert. Die Fermentationsbrühe (sowohl die vorgelegte als auch die zugeführte) hatte einen Gehalt an ortho-Aminobenzoesäure von 91 g/L, die Salzsäure hatte einen Massengehalt von 17 %. Die Dosierung wurde über 2 h durchgeführt, wobei der pH-Wert zwischen 2,3 (kurzzeitig zu Beginn der Dosierung) und 3,5 (über die gesamte restliche Dosierzeit) lag (Schritt **(III)** des erfindungsgemäßen Verfahrens).

Nach einer Nachrührzeit von 1 h wurde das Kristallisat durch Filtration isoliert **(Schritt (IV)** des erfindungsgemäßen Verfahrens) und mit Wasser gewaschen **(Schritt (V)** des erfindungsgemäßen Verfahrens).

Der Filterwiderstand wurde zu α = 6 10⁺¹⁰ 1/m² bestimmt, die Restfeuchte im Kuchen lag bei 30,5 % und die isolierte Ausbeute betrug 89 %. Die Kristallgröße wurde anhand von mikroskopischen Bildern zu 400 µm abgeschätzt.

Das Kristallisat lag in der Modifikation I vor. Der Gehalt des Kristallisats an ortho-Aminobenzoesäure lag bei 98,5 %, der Aschegehalt betrug 0,03 %.

### Beispiel 5 (semi-kontinuierliche Durchführung von Schritt (III) - kontinuierliche Zuführung von Salzsäure und Fermentationsbrühe, absatzweise Entnahme von Suspension zu einer Suspension an Impfkristallen)

Beispiel 5 wurde durchgeführt wie Beispiel 4 mit dem Unterschied, dass nicht nur die Edukte Fermentationsbrühe und Salzsäure kontinuierlich zudosiert wurden, sondern auch Suspension absatzweise entnommen wurde, was einer vollkontinuierlichen Fahrweise nahe kommt. Die Dosierung der Edukte Salzsäure und Fermentationsbrühe erfolgte so, dass sich eine Verweilzeit der Kristalle im Reaktor von ca. 45 min ergab. Der pH-Wert variierte zwischen 3,2 und 3,5. Alle 15 min wurde Suspension entnommen (jeweils 60 ml bei 200 ml Gesamtansatzgröße) und charakterisiert.

Die Restfeuchte im Filterkuchen lag bei 1 % und die isolierte Ausbeute betrug 91 %. Die Kristallgröße wurde anhand von mikroskopischen Bildern zu 400 µm abgeschätzt.

Das Kristallisat lag in der Modifikation I vor. Der Gehalt des Kristallisats an ortho-Aminobenzoesäure lag bei 99 %, der Aschegehalt betrug 0,02 %.

## Patentansprüche

1. Verfahren zur Herstellung von ortho-Aminobenzoesäure oder eines ortho-Aminobenzoesäurederivats, umfassend die Schritte:
(I) Fermentation eines Rohstoffes, der wenigstens eine fermentierbare Kohlenstoff-haltige Verbindung umfasst unter Verwendung von Mikroorganismen einer Art ausgewählt aus der Gruppe bestehend aus *Escherichia coli, Pseudomonas putida* und *Corynebacterium glutamicum,* wobei eine ortho-Aminobenzoat und/oder ortho-Aminobenzoesäure umfassende Fermentationsbrühe erhalten wird;
(II) optionale **Vorbehandlung** der in Schritt (I) erhaltenen Fermentationsbrühe umfassend
(1) **Abtrennung des Mikroorganismus** aus der in Schritt (I) erhaltenen Fermentationsbrühe ohne pH-Wert-Anpassung, wobei eine an Mikroorganismen abgereicherte Fermentationsbrühe erhalten wird,
und/oder
(2) **Entfärbung** der in Schritt (I) erhaltenen Fermentationsbrühe oder, bei Durchführung von Schritt (II) (1), der in Schritt (II) (1) erhaltenen an Mikroorganismen abgereicherten Fermentationsbrühe, ohne pH-Wert-Anpassung;
(III) einstufige Behandlung der in Schritt (I) oder Schritt (II) (1) oder Schritt (II) (2) erhaltenen Fermentationsbrühe in einem Reaktor mit Säure so, dass sich ortho-Aminobenzoesäure aus der Fermentationsbrühe abscheidet;
(IV) Isolierung der in Schritt (III) abgeschiedenen ortho-Aminobenzoesäure, wobei Mutterlauge zurückbleibt;
(V) optionale weitere Reinigung der in Schritt (IV) gewonnenen ortho-Aminobenzoesäure, bevorzugt durch Waschen mit Wasser;
(VI) optionale weitere Umsetzung der in Schritt (IV) oder Schritt (V) erhaltenen ortho-Aminobenzoesäure zu einem ortho-Aminobenzosäurederivat;
wobei Schritt (III) so durchgeführt wird, dass Impfkristalle von ortho-Aminobenzoesäure zugegen sind und die Anwesenheit der Impfkristalle derart erreicht wird, dass die Fermentationsbrühe und die Säure zu einer im Reaktor vorgelegten Suspension an Impfkristallen gegeben werden, wobei die in der Suspension vorgelegten Impfkristalle zu mindestens 90 %, bezogen auf die Gesamtmasse aller in der Suspension vorgelegten
Impfkristalle, aus der Modifikation Form I der ortho-Aminobenzoesäure, entsprechend der Nomenklatur in Ojala, W. H., Etter, M. C., J. Am. Chem. Soc. 1992, 114, 10288 - 10293, bestehen,
und wobei ein Aminobenzoesäurederivat ein Produkt bezeichnet, welches durch weitere chemische Umsetzung von Aminobenzoesäure erhalten wird.

2. Verfahren gemäß Anspruch 1, bei welchem in Schritt (III) der pH-Wert der durch die Behandlung mit Säure resultierenden Mischung auf einen Wert im Bereich von 3,0 bis 4,7 eingestellt wird.

3. Verfahren gemäß Anspruch 1, bei dem die Säurebehandlung in Schritt (III) so durchgeführt wird, dass der pH-Wert der resultierenden Mischung dem des isoelektrischen Punkts des ortho-Isomers von Aminobenzoesäure entspricht.

4. Verfahren gemäß einem der vorstehenden Ansprüche, bei dem die in Schritt (III) eingesetzte Säure Salzsäure, Schwefelsäure und/oder Phosphorsäure umfasst.

5. Verfahren gemäß dem Anspruch 4, bei dem die in Schritt (III) eingesetzte Säure eine Mischung aus Salzsäure und einem Teil der in Schritt (IV) erhaltenen Mutterlauge ist.

6. Verfahren gemäß einem der vorstehenden Ansprüche, bei dem in Schritt (III) die Fermentationsbrühe und die Säure dem Reaktor über räumlich getrennte Zuführeinrichtungen zugeführt werden.

7. Verfahren gemäß einem der vorstehenden Ansprüche, bei dem in Schritt (III) bei kontinuierlicher Durchführung des Schrittes (III)
▪ die Säure mit einer solchen Dosiergeschwindigkeit und
▪ die Fermentationsbrühe mit einer solchen Dosiergeschwindigkeit dem Reaktor zugeführt werden und eine Suspension von Aminobenzoesäure in Mutterlauge dem Reaktor absatzweise oder kontinuierlich so entnommen wird, dass sich eine Verweilzeit der Suspension im Reaktor von ¼ h bis 10 h ergibt
oder
bei diskontinuierlicher Durchführung des Schrittes (III) die einstufige Säurebehandlung für einen Zeitraum von ¼ h bis 10 h betrieben wird.

8. Verfahren gemäß einem der vorstehenden Ansprüche, bei dem die in Schritt (I) eingesetzten Mikroorganismen *Corynebacterium glutamicum* ATTC 13032 umfassen.

9. Verfahren gemäß Anspruch 8, bei dem die in Schritt (I) eingesetzten Mikroorganismen nur aus *Corynebacterium glutamicum* ATTC 13032 bestehen.

10. Verfahren gemäß einem der Ansprüche 1 bis 9, bei dem die Suspension an Impfkristallen eine Suspension der Impfkristalle in einem Teil der in Schritt (I) oder Schritt (II) erhaltenen Fermentationsbrühe ist.

11. Verfahren gemäß Anspruch 10, wobei die Impfkristalle in 1,0 Massen-% bis 20 Massen-% der in Schritt (III) insgesamt eingesetzten Fermentationsbrühe suspendiert werden.

12. Verfahren gemäß einem der Ansprüche 1 bis 11, bei dem Schritt (III) diskontinuierlich durchgeführt wird.

13. Verfahren gemäß einem der Ansprüche 1 bis 11, bei dem Schritt (III) kontinuierlich durchgeführt wird und die Anwesenheit von Impfkristallen von ortho-Aminobenzoesäure in Schritt (III) derart erreicht wird, dass Fermentationsbrühe und Säure kontinuierlich dem Reaktor zugeführt werden und eine Suspension von ortho-Aminobenzoesäure in Mutterlauge dem Reaktor kontinuierlich entnommen wird, wobei die Zufuhr an Fermentationsbrühe und Säure sowie die Entnahme an Suspension so eingestellt werden, dass in dem im Reaktor befindlichen Teil der Reaktionsmischung stets Kristalle von ortho-Aminobenzoesäure vorhanden sind.

14. Verfahren gemäß einem der vorstehenden Ansprüche, bei dem Schritt (VI) durchgeführt wird und eine der folgenden Umsetzungen umfasst:
(VI-1) Decarboxlierung zu Anilin;
(VI-2) Decarboxylierung gefolgt von säurekatalysierter Reaktion mit Formaldehyd unter Bildung von Di- und Polyaminen der Diphenylmethanreihe;
(VI-3) Decarboxylierung gefolgt von säurekatalysierter Reaktion mit Formaldehyd, gefolgt von Umsetzung mit Phosgen unter Bildung von Di- und Polyisocyanaten der Diphenylmethanreihe;
(VI-4) Umsetzung zu einer Azoverbindung;
(VI-5) Umsetzung zu Amiden;
(VI-6) Umsetzung zu leitenden Polymeren.

15. Verfahren gemäß einem der vorstehenden Ansprüche, bei welchem der Rohstoff in Schritt (I) ausgewählt ist aus der Gruppe bestehend aus Stärkehydrolysat, Zuckerrohrsaft und Zuckerrübensaft.

## Claims

1. Process for preparing ortho-aminobenzoic acid or an ortho-aminobenzoic acid derivative, comprising the steps:
(I) fermentation of a raw material which comprises at least one fermentable carbon-containing compound by using microorganisms of a species selected from the group consisting of *Escherichia coli, Pseudomonas putida* and *Corynebacterium glutamicum,* wherein an ortho-aminobenzoate- and/or ortho-aminobenzoic-acid-comprising fermentation broth is obtained;
(II) optional **pretreatment** of the fermentation broth obtained in step (I), comprising
(1) **removal of the microorganism** from the fermentation broth obtained in step (I) without pH adjustment, wherein a microorganism-depleted fermentation broth is obtained,
and/or
(2) **decolouration** of the fermentation broth obtained in step (I) or, when carrying out step (II) (1), of the microorganism-depleted fermentation broth obtained in step (II) (1), without pH adjustment;
(III) one-step treatment of the fermentation broth obtained in step (I) or step (II) (1) or step (II) (2) in a reactor with acid such that ortho-aminobenzoic acid is precipitated from the fermentation broth;
(IV) isolation of the ortho-aminobenzoic acid precipitated in step (III), wherein mother liquor remains;
(V) optional further purification of the ortho-aminobenzoic acid gained in step (IV), preferably by washing with water;
(VI) optional further conversion of the ortho-aminobenzoic acid obtained in step (IV) or step (V) to form an ortho-aminobenzoic acid derivative;
wherein step (III) is carried out such that seed crystals of ortho-aminobenzoic acid are present and the presence of the seed crystals is achieved such that the fermentation broth and the acid are added to a suspension of seed crystals that has been initially charged in the reactor, wherein the seed crystals initially charged in the suspension consist, to an extent of at least 90% based on the total mass of all seed crystals initially charged in the suspension, of the form I modification of the ortho-aminobenzoic acid, according to the nomenclature in Ojala, W. H., Etter, M. C., J. Am. Chem. Soc. 1992, 114, 10288 - 10293, and wherein an aminobenzoic acid derivative means a product which is obtained by further chemical conversion of aminobenzoic acid.

2. Process according to Claim 1, in which, in step (III), the pH of the resulting mixture from the treatment with acid is set to a level in the range from 3.0 to 4.7.

3. Process according to Claim 1, in which the acid treatment in step (III) is carried out such that the pH of the resulting mixture corresponds to that of the isoelectric point of the ortho isomer of aminobenzoic acid.

4. Process according to any of the preceding claims, in which the acid used in step (III) comprises hydrochloric acid, sulfuric acid and/or phosphoric acid.

5. Process according to Claim 4, in which the acid used in step (III) is a mixture of hydrochloric acid and a portion of the mother liquor obtained in step (IV) .

6. Process according to any of the preceding claims, in which, in step (III), the fermentation broth and the acid are fed to the reactor via feeding devices spaced apart.

7. Process according to any of the preceding claims, in which, in step (III),
with continuous performance of step (III), there is feeding of
▪ the acid at such a metering rate and
▪ the fermentation broth at such a metering rate to the reactor and withdrawal of a suspension of aminobenzoic acid in mother liquor from the reactor at intervals or in a continuous manner such that a residence time of the suspension in the reactor of from ¼ h to 10 h arises
or
with discontinuous performance of step (III), the one-stage acid treatment is run over a period of from ¼ h to 10 h.

8. Process according to any of the preceding claims, in which the microorganisms used in step (I) comprise *Corynebacterium glutamicum* ATTC 13032.

9. Process according to Claim 8, in which the microorganisms used in step (I) consist only of *Corynebacterium glutamicum* ATTC 13032.

10. Process according to any of Claims 1 to 9, in which the suspension of seed crystals is a suspension of the seed crystals in a portion of the fermentation broth obtained in step (I) or step (II).

11. Process according to Claim 10, wherein the seed crystals are suspended in 1.0% by mass to 20% by mass of the fermentation broth used altogether in step (III).

12. Process according to any of Claims 1 to 11, in which step (III) is carried out discontinuously.

13. Process according to any of Claims 1 to 11, in which step (III) is carried out continuously and the presence of seed crystals of ortho-aminobenzoic acid in step (III) is achieved such that fermentation broth and acid are added continuously to the reactor and a suspension of ortho-aminobenzoic acid in mother liquor is continuously withdrawn from the reactor, wherein the feeding of fermentation broth and acid and also the withdrawal of suspension are set such that, in the part of the reaction mixture that is present in the reactor, crystals of ortho-aminobenzoic acid are always present.

14. Process according to any of the preceding claims, in which step (VI) is carried out and comprises one of the following conversions:
(VI-1) decarboxylation to form aniline;
(VI-2) decarboxylation followed by acid-catalysed reaction with formaldehyde to form di- and polyamines of the diphenylmethane series;
(VI-3) decarboxylation followed by acid-catalysed reaction with formaldehyde, followed by reaction with phosgene to form di- and polyisocyanates of the diphenylmethane series;
(VI-4) conversion to an azo compound;
(VI-5) conversion to amides;
(VI-6) conversion to conductive polymers.

15. Process according to any of the preceding claims, in which the raw material in step (I) is selected from the group consisting of starch hydrolysate, sugarcane juice and sugar beet juice.

## Revendications

1. Procédé de préparation d'acide ortho-aminobenzoique ou d'un dérivé de l'acide ortho-aminobenzoïque, comprenant les étapes :
(I) fermentation d'une matière première qui comprend au moins un composé carboné fermentable, par utilisation de microorganismes d'une espèce choisie dans le groupe consistant en *Escherichia coli, Pseudomonas putida* et *Corynebacterium glutamicum,* avec obtention d'un bouillon de fermentation contenant un ortho-aminobenzoate et/ou de l'acide ortho-aminobenzoïque ;
(II) **prétraitement** éventuel du bouillon de fermentation obtenu dans l'étape (I), comprenant
(1) **la séparation du microorganisme** du bouillon de fermentation obtenu dans l'étape (I) sans ajustement du pH, avec obtention d'un bouillon de fermentation appauvri en microorganismes,
et/ou
(2) **décoloration** du bouillon de fermentation obtenu dans l'étape (I) ou, lors de la mise en œuvre de l'étape (II) (1), du bouillon de fermentation appauvri en microorganismes et obtenu dans l'étape (II) (1), sans ajustement du pH ;
(III) traitement par un acide en une étape du bouillon de fermentation obtenu dans l'étape (I) ou dans l'étape (II) (1) ou dans l'étape (II)(2) dans un réacteur de façon que l'acide ortho-aminobenzoïque se sépare du bouillon de fermentation ;
(IV) isolement de l'acide ortho-aminobenzoïque séparé dans l'étape (III), avec obtention d'une lessive-mère résiduelle ;
(V) purification supplémentaire éventuelle de l'acide ortho-aminobenzoïque obtenu dans l'étape (IV), de préférence par lavage à l'eau ;
(VI) réaction supplémentaire éventuelle de l'acide ortho-aminobenzoïque obtenu dans l'étape (IV) ou dans l'étape (V), pour obtenir un dérivé de l'acide ortho-aminobenzoïque ;
dans lequel l'étape (III) est mise en œuvre de telle sorte que des germes cristallins de l'acide ortho-aminobenzoïque soient présents, et que l'on en arrive à la présence des germes cristallins par le fait que le bouillon de fermentation et l'acide sont ajoutés à une suspension de germes cristallins, déjà en place dans le réacteur, les germes cristallins déjà en place dans la suspension étant, à raison d'au moins 90 % par rapport à la masse totale de tous les germes cristallins déjà en place dans la suspension, constitués de la modification I de l'acide ortho-aminobenzoïque, correspondant à la nomenclature d'Ojala, W. H., Etter, M. C., J. Am. Chem. Soc. 1992, 114, 10288 - 10293,
et un dérivé de l'acide aminobenzoïque désignant un produit qui est obtenu par une réaction chimique supplémentaire de l'acide aminobenzoïque.

2. Procédé selon la revendication 1, dans lequel, dans l'étape (III), le pH du mélange obtenu par traitement par un acide est ajusté à une valeur dans la plage de 3,0 à 4,7.

3. Procédé selon la revendication 1, dans lequel le traitement par un acide de l'étape (III) est mis en œuvre de telle sorte que le pH du mélange obtenu corresponde à celui du point isoélectrique de l'isomère ortho de l'acide aminobenzoïque.

4. Procédé selon l'une des revendications précédentes, dans lequel l'acide utilisé dans l'étape (III) comprend l'acide chlorhydrique, l'acide sulfurique et/ou l'acide phosphorique.

5. Procédé selon la revendication 4, dans lequel l'acide utilisé dans l'étape (III) est un mélange d'acide chlorhydrique et d'une partie de la lessive-mère obtenue dans l'étape (IV).

6. Procédé selon l'une des revendications précédentes, dans lequel, dans l'étape (III), le bouillon de fermentation et l'acide sont envoyés dans le réacteur par des dispositifs d'amenée spatialement séparés.

7. Procédé selon l'une des revendications précédentes, dans lequel, dans l'étape (III), en présence d'une mise en œuvre continue de l'étape (III), on amène au réacteur
▪ l'acide, à une certaine vitesse de dosage et
▪ le bouillon de fermentation avec une certaine vitesse de dosage, et on prélève du réacteur, par lots ou en continu, une suspension d'acide aminobenzoique dans la lessive-mère, les vitesse de dosage étant telles qu'il en résulte un temps de séjour de la suspension dans le réacteur de ¼ d'heure à 10 heures
ou
dans le cas d'une mise en œuvre discontinue de l'étape (III), le traitement à l'acide en une étape est réalisé pendant une durée de ¼ d'heure à 10 heures.

8. Procédé selon l'une des revendications précédentes, dans lequel les microorganismes utilisés dans l'étape (I) comprennent *Corynebacterium glutamicum* ATTC 13032.

9. Procédé selon la revendication 8, dans lequel les microorganismes utilisés dans l'étape (I) ne sont constitués que de *Corynebacterium glutamicum* ATTC 13032.

10. Procédé selon l'une des revendications 1 à 9, dans lequel la suspension de germes cristallins est une suspension des germes cristallins dans une partie du bouillon de fermentation obtenu dans l'étape (I) ou dans l'étape (II).

11. Procédé selon la revendication 10, dans lequel les germes cristallins sont mis en suspension dans 1,0 % en masse à 20 % en masse du bouillon de fermentation utilisé en totalité dans l'étape (III).

12. Procédé selon l'une des revendications 1 à 11, dans lequel l'étape (III) est mise en œuvre d'une manière discontinue.

13. Procédé selon l'une des revendications 1 à 11, dans lequel l'étape (III) est mise en œuvre d'une manière continue, et on atteint la présence de germes cristallins d'acide ortho-aminobenzoïque dans l'étape (III) en amenant le bouillon de fermentation et l'acide en continu au réacteur et en prélevant en continu du réacteur une suspension d'acide ortho-aminobenzoïque dans la lessive-mère, l'amenée du bouillon de fermentation et de l'acide, ainsi que le prélèvement de la suspension, étant ajustés de façon que des cristaux d'acide ortho-aminobenzoïque soient toujours présents dans la partie du mélange réactionnel se trouvant dans le réacteur.

14. Procédé selon l'une des revendications précédentes, dans lequel l'étape (VI) est mise en œuvre et comprend l'une des réactions suivantes :
(VI-1) décarboxylation en aniline ;
(VI-2) décarboxylation suivie d'une réaction catalysée par un acide avec du formaldéhyde, avec formation de di- et de polyamines de la série du diphénylméthane ;
(VI-3) décarboxylation suivie d'une réaction catalysée par un acide avec du formaldéhyde, suivie de la conversion du phosgène avec formation de di- et de polyisocyanate de la série du diphénylméthane ;
(VI-4) conversion en un composé azoïque ;
(VI-5) conversion en amides ;
(VI-6) conversion en des polymères conducteurs.

15. Procédé selon l'une des revendications précédentes, dans lequel la matière première de l'étape (I) est choisie dans le groupe consistant en un hydrolysat d'amidon, le vesou et le jus de betterave.
